# FASCICULE DE BREVET EUROPEEN

(11) **EP 3 606 583 B1**
(45) Date de publication et mention de la délivrance du brevet: **01.11.2023**
(21) Numéro de dépôt: 18718599.6
(22) Date de dépôt: 29.03.2018
(51) Int. Cl.: A61M 5/31, A61K 31/728, A61K 9/00

(54) **SERINGUE À CENTRE DE GRAVITÉ OPTIMISÉ**
SPRITZE MIT OPTIMIERTEM SCHWERPUNKT
SYRINGE WITH OPTIMISED CENTRE OF GRAVITY

(30) Priorité: 04.04.2017 FR 1752876
(43) Date de publication de la demande: 12.02.2020
(73) Titulaire: Laboratoires Vivacy, 75116 Paris (FR)
(72) Inventeur: HEIM, Ludovic, 74960 Cran Gevrier (FR); GANTIN, Denis, 74130 Ayze (FR)
(74) Mandataire: INNOV-GROUP
(86) Numéro de dépôt international: PCT/FR2018/050780
(87) Numéro de publication internationale: WO 2018/185406

(56) Documents cités:
- EP-A1- 2 574 357
- WO-A1-2014/033143
- US-A- 3 705 584
- US-A- 4 132 231
- US-A1- 2014 039 456

## Description

### Domaine technique

L'invention se rapporte au domaine technique des seringues.

L'invention trouve notamment son application dans l'injection de gels viscoélastiques à base d'acide hyaluronique, dans le domaine de l'anti-âge et de l'esthétique médicale.

### Etat de la technique antérieure

Une seringue connue de l'état de la technique, comporte :
- un corps de seringue, destiné à recevoir un produit à injecter ; le corps de seringue présentant une première extrémité, destinée à être équipée d'un porte-aiguille, et une seconde extrémité opposée ;
- des ailettes de préhension, agencées à la seconde extrémité du corps de seringue ;
- une tige de piston, montée coulissante à l'intérieur du corps de seringue, la tige de piston présentant une première extrémité et une seconde extrémité opposée ;
- un butoir, agencé à la première extrémité de la tige de piston, et présentant une position de fin de course à l'intérieur du corps de seringue ;
- un poussoir, agencé à la seconde extrémité de la tige de piston.

Une telle seringue de l'état de la technique n'est pas entièrement satisfaisante dans le cas où le produit à injecter nécessite une pression importante à exercer sur le poussoir. C'est notamment le cas lorsque le produit à injecter est un gel viscoélastique à base d'acide hyaluronique, où il est généralement nécessaire d'appliquer une force supérieure à 100 N. Le médecin est donc susceptible de ressentir un certain inconfort, voire des douleurs, en utilisant de manière répétée une telle seringue de l'état de la technique. Nous emploierons par la suite le terme « médecin », étant entendu qu'il désigne tout utilisateur de la seringue.

En outre, le produit peut être injecté au niveau des rides d'un patient, plus particulièrement sous les yeux. Une telle injection requiert une excellente stabilité de la seringue afin d'obtenir la précision souhaitée (de l'ordre de ±1 mm) et atteindre les rides du patient de manière effective et sécurisée.

Le document EP 2 574 357 A1 divulgue un dispositif d'injection motorisé adapté pour recevoir un corps de seringue. Le boîtier est équipé d'un moteur couplé à une tige de piston, la tige de piston étant agencée pour expulser le produit à injecter. Le document US 4 132 231 A divulgue un dispositif d'injection comportant un corps de seringue. Le dispositif comporte deux pistons reliés à un poussoir sur lequel sont ajoutés des moyens de lestage. Le document US 2014/039456 A1 divulgue un dispositif d'injection d'une composition pharmaceutique dans l'oeil. Le document WO 2014/033143 A1 divulgue un ensemble de protection d'un dispositif médical. Le document US 3 705 584 divulgue un ensemble pour stabiliser une seringue.

### Exposé de l'invention

L'invention vise à remédier en tout ou partie aux inconvénients précités. A cet effet, l'invention a pour objet une seringue, définie selon la revendication 1 annexée.

Ainsi, une telle seringue selon l'invention possède une excellente stabilité grâce à un tel positionnement du centre de gravité, ce qui autorise une grande précision lors de l'injection. En effet, le centre de gravité de la seringue selon l'invention est décalé par rapport à l'état de la technique, en étant plus proche de la main du médecin qui va exercer une pression sur le poussoir. Cela permet également d'améliorer le confort du médecin pendant une utilisation répétée.

En outre, une telle seringue selon l'invention favorise la répétabilité du positionnement de la seringue dans la main du médecin, ce qui contribue à améliorer la précision lors de l'injection. En effet, un tel positionnement du centre de gravité facilite le basculement de la seringue vers la paume de la main du médecin. Les doigts du médecin sur lesquels reposent les ailettes de préhension forment une surface d'appui qui est une base de sustentation de la seringue. La droite d'action du poids de la seringue doit être en dehors de la base de sustentation afin que la seringue puisse basculer vers la paume de la main du médecin. Le fait que le centre de gravité de la seringue est à l'extérieur du corps de seringue lorsque le butoir est dans la position de fin de course permet d'obtenir un centre de gravité éloigné des ailettes de préhension, et par là-même un centre de gravité éloigné de la base de sustentation, ce qui facilite ainsi ultérieurement le basculement de la seringue car la droite d'action du poids de la seringue peut se retrouver plus rapidement en dehors de la base de sustentation.

La seringue selon l'invention peut comporter une ou plusieurs des caractéristiques suivantes.

Selon l'invention, le centre de gravité est situé au sein de la tige de piston, entre les ailettes de préhension et le poussoir, lorsque le butoir est dans la position de fin de course.

Ainsi, un avantage procuré est d'obtenir un compromis entre stabilité et maniabilité de la seringue, ce qui peut être utile pour certaines applications.

Selon une caractéristique de l'invention, le centre de gravité est situé à proximité de la seconde extrémité de la tige de piston lorsque le butoir est dans la position de fin de course.

Ainsi, un avantage procuré est de rapprocher le centre de gravité de la seringue de la main du médecin afin d'accroître la stabilité de la seringue lorsque la pression exercée sur le poussoir est importante.

En outre, un tel positionnement du centre de gravité favorise le basculement de la seringue vers la paume de la main du médecin afin d'obtenir une excellente répétabilité du positionnement de la seringue dans la main du médecin.

Selon l'invention, le corps de seringue, les ailettes de préhension, la tige de piston et le butoir possèdent une masse totale ; et la valeur seuil de la masse du poussoir est déterminée en fonction de la masse totale.

Ainsi, un avantage procuré est de pouvoir positionner le centre de gravité de la seringue en tenant compte de différentes configurations possibles de la seringue (géométrie, type de matériau utilisé...).

Selon une caractéristique de l'invention, la valeur seuil de la masse du poussoir est 4 grammes.

Ainsi, il a été constaté que cette valeur seuil permet d'obtenir un centre de gravité situé à l'extérieur du corps de seringue lorsque le butoir est dans la position de fin de course, et ce pour des configurations classiques de seringues.

Selon une caractéristique de l'invention, le poussoir présente une surface de préhension, de préférence elliptique, possédant une aire comprise entre 800 mm² et 900 mm².

Ainsi, un avantage procuré est d'augmenter la surface de préhension du poussoir par rapport à l'état de la technique (de l'ordre de 80% par rapport à des configurations classiques de seringue), ce qui permet notamment d'envisager d'actionner le poussoir avec la paume de la main (et non simplement avec le pouce) sans nuire à la stabilité de la seringue.

La surface de préhension du poussoir est de préférence elliptique afin d'éviter la présence d'arêtes vives pouvant blesser le médecin.

En outre, une augmentation de la surface de préhension du poussoir confère une plus grande liberté dans les angles d'injection.

Selon une caractéristique de l'invention, les ailettes de préhension présentent une surface de préhension possédant une aire comprise entre 700 mm² et 800 mm², de préférence comprise entre 700 mm² et 750 mm².

Ainsi, un avantage procuré est d'augmenter la surface de préhension des ailettes de préhension par rapport à l'état de la technique (de l'ordre de 90% par rapport à des configurations classiques de seringue). Il en résulte une amélioration de la stabilité de la seringue en augmentant la surface de contact avec les doigts du médecin. Par ailleurs, une telle augmentation de la surface de préhension (et donc de sa masse) est compensée par une masse adaptée du poussoir afin de maintenir un centre de gravité situé à l'extérieur du corps de seringue lorsque le butoir est dans la position de fin de course.

Selon une caractéristique de l'invention, les ailettes de préhension présentent une surface de préhension concave, orientée vers la première extrémité du corps de seringue.

Ainsi, un avantage procuré par la concavité de la surface de préhension est de pouvoir éviter un glissement latéral des doigts du médecin à la surface de préhension, et par là-même sécuriser le geste d'injection.

Selon une caractéristique de l'invention, les ailettes de préhension s'étendent suivant un axe longitudinal, et présentent un profil transversal formant une surface convexe.

Ainsi, un avantage procuré par un tel profil transversal est de faciliter le basculement de la seringue vers la paume de la main du médecin grâce à la convexité de la surface.

Selon une caractéristique de l'invention, le corps de seringue, les ailettes de préhension, la tige de piston et le poussoir sont réalisés au moins partiellement dans un premier matériau plastique, de préférence le polycarbonate ; le poussoir présente une surface de préhension au moins partiellement réalisée dans un second matériau plastique, différent du premier matériau plastique, et possédant une dureté comprise entre 30 Shore A et 70 Shore A, de préférence comprise entre 50 Shore A et 70 Shore A ; les ailettes de préhension présentent une surface de préhension au moins partiellement réalisée dans le second matériau plastique.

Ainsi, tel second matériau plastique est suffisamment mou pour obtenir une excellente accroche. Par ailleurs, l'utilisation de deux matériaux plastiques distincts permet de rendre attrayante la seringue selon l'invention.

### Brève description des dessins

D'autres caractéristiques et avantages apparaîtront dans l'exposé détaillé de différents modes de réalisation de l'invention, l'exposé étant assorti d'exemples et de référence aux dessins joints.
Figure 1 est une vue schématique en perspective d'une seringue selon l'invention en position verticale, en l'absence de porte-aiguille.
Figure 2 est une vue schématique analogue à la figure 1, illustrant la seringue en position horizontale.
Figure 3 est une vue schématique en perspective d'une seringue selon l'invention, en position horizontale dans la main d'un médecin, et équipée d'un porte-aiguille.
Figure 4 est une vue schématique en perspective d'ailettes de préhension équipant une seringue selon l'invention.
Figure 5 est une vue schématique en coupe longitudinale des ailettes de préhension illustrées à la figure 4. Par « longitudinale », on entend une direction suivant l'axe longitudinal des ailettes de préhension.
Figure 6 est une vue schématique en coupe transversale des ailettes de préhension illustrées à la figure 4. Par « transversale », on entend suivant une direction perpendiculaire à l'axe longitudinal des ailettes de préhension.
Figures 7 à 9 sont des vues schématiques en perspective d'une seringue selon l'invention dans la main d'un médecin, et illustrant différentes positions des doigts permettant l'injection.
Figure 10 est une vue schématique d'un poussoir équipant une seringue selon l'invention, illustrant la surface du poussoir orientée vers les ailettes de préhension.
Figure 11 est une vue schématique d'un profil de poussoir équipant une seringue selon l'invention.

### Exposé détaillé des modes de réalisation

Les éléments identiques ou assurant la même fonction porteront les mêmes références pour les différents modes de réalisation, par souci de simplification.

Un objet de l'invention est une seringue 1, comportant :
- un corps 2 de seringue, destiné à recevoir un produit à injecter ; le corps 2 de seringue présentant une première extrémité 20, destinée à être équipée d'un porte-aiguille 3, et une seconde extrémité 21 opposée ;
- des ailettes 4 de préhension, agencées à la seconde extrémité 21 du corps 2 de seringue ;
- une tige 5 de piston, montée coulissante à l'intérieur du corps 2 de seringue, la tige 5 de piston présentant une première extrémité 50 et une seconde extrémité 51 opposée ;
- un butoir 6, agencé à la première extrémité 50 de la tige 5 de piston, et présentant une position de fin de course à l'intérieur du corps 2 de seringue ;
- un poussoir 7, agencé à la seconde extrémité 51 de la tige 5 de piston ;
la seringue étant remarquable en ce que le poussoir 7 possède une masse supérieure ou égale à une valeur seuil au-delà de laquelle la seringue 1 possède un centre de gravité situé à l'extérieur du corps 2 de seringue lorsque le butoir 6 est dans la position de fin de course.

### Corps de seringue

Le corps 2 de seringue est de préférence cylindrique. Le produit à injecter est de préférence un gel viscoélastique à base d'acide hyaluronique.

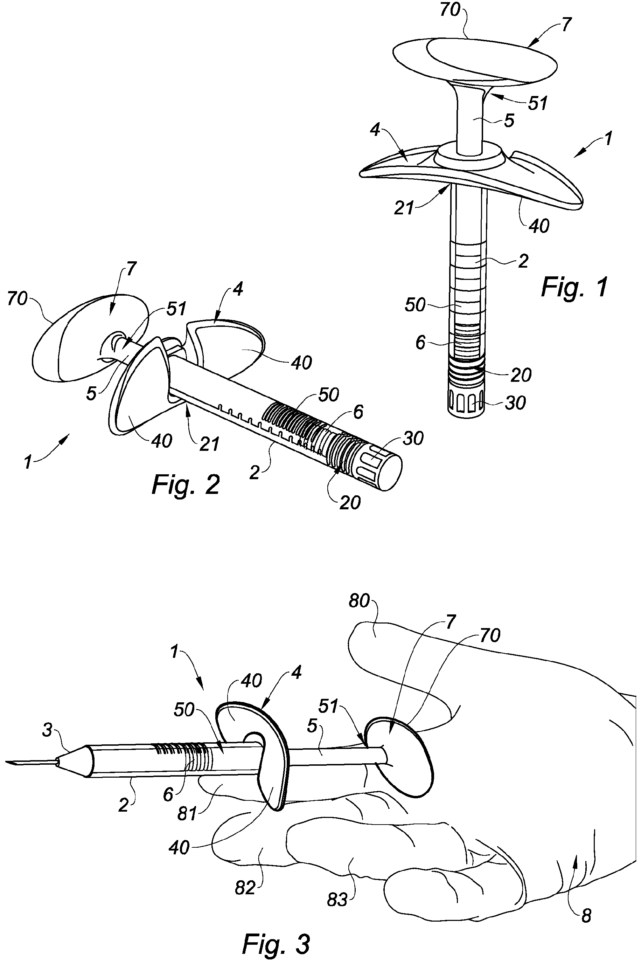

Les première et seconde extrémités 20, 21 du corps 2 de seringue sont ouvertes. La première extrémité 20 du corps 2 de seringue est ouverte pour recevoir un porte-aiguille 3. En l'absence de porte-aiguille 3, comme illustré aux figures 1 et 2, la première extrémité 20 du corps 2 de seringue est préférentiellement obstruée par un capuchon 30. La seconde extrémité 21 du corps 2 de seringue est ouverte pour recevoir la tige 5 de piston.

Avantageusement, le corps 2 de seringue est réalisé dans un premier matériau plastique. Le premier matériau plastique est de préférence le polycarbonate.

### Tige de piston et butoir

Avantageusement, la tige 5 de piston est réalisée dans le premier matériau plastique.

Le butoir 6 forme une tête de piston. Lorsque le butoir 6 est dans la position de fin de course, la tige 5 de piston est avantageusement en saillie du corps 2 de seringue sur une distance comprise entre 20 mm et 30 mm.

### Poussoir

Le poussoir 7 possède une masse supérieure ou égale à une valeur seuil au-delà de laquelle le centre de gravité de la seringue 1 est situé au sein de la tige 5 de piston, entre les ailettes 4 de préhension et le poussoir 7, lorsque le butoir 6 est dans la position de fin de course.

Le poussoir 7 possède avantageusement une masse supérieure ou égale à une valeur seuil au-delà de laquelle le centre de gravité de la seringue 1 est situé à proximité de la seconde extrémité 51 de la tige 5 de piston lorsque le butoir 6 est dans la position de fin de course.

Le corps 2 de seringue, les ailettes 4 de préhension, la tige 5 de piston et le butoir 6 possèdent une masse totale. La valeur seuil de la masse du poussoir 7 est déterminée en fonction de la masse totale de sorte que la seringue 1 possède un centre de gravité situé à l'extérieur du corps 2 de seringue lorsque le butoir 6 est dans la position de fin de course.

La valeur seuil de la masse du poussoir 7 est avantageusement 4 grammes. La masse du poussoir 7 est avantageusement inférieure ou égale à 10 grammes afin de ne pas alourdir inutilement la seringue 1. La masse du poussoir 7 est avantageusement comprise entre 4 grammes et 5 grammes.

Le poussoir 7 présente une surface de préhension 70, de préférence elliptique, possédant avantageusement une aire comprise entre 800 mm² et 900 mm².

Avantageusement, le poussoir 7 est réalisé au moins partiellement dans le premier matériau plastique. Avantageusement, le poussoir 7 présente une surface de préhension 70 au moins partiellement réalisée dans un second matériau plastique, différent du premier matériau plastique. Le second matériau plastique possède avantageusement une dureté comprise entre 30 Shore A et 70 Shore A, de préférence comprise entre 50 Shore A et 70 Shore A. A titre d'exemples non limitatifs, le second matériau plastique peut être un thermoplastique de type SEBS i.e. polystyrène-b-poly(éthylène-butylène)-b-polystyrène, ou le silicone.

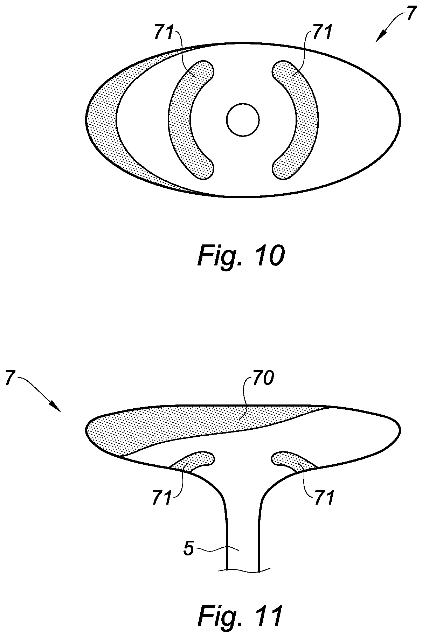

Comme illustré aux figures 10 et 11, le poussoir 7 présente avantageusement au moins une surface de préhension additionnelle 71, opposée à la surface de préhension 70. La ou les surfaces de préhension additionnelles 71 sont avantageusement au moins partiellement réalisées dans le second matériau plastique. De telles surfaces de préhension additionnelles 71 permettent de faciliter la réaspiration du produit à injecter par le médecin.

### Ailettes de préhension

Les ailettes 4 de préhension présentent une surface de préhension 40 possédant avantageusement une aire comprise entre 700 mm² et 800 mm², de préférence comprise entre 700 mm² et 750 mm². Les ailettes 4 de préhension forment des parties latérales en saillie du corps 2 de seringue, et en forme d'aile. Les ailettes 4 de préhension sont avantageusement monobloc.

Les ailettes 4 de préhension présentent avantageusement une surface de préhension 40 concave, orientée vers la première extrémité 20 du corps 2 de seringue.

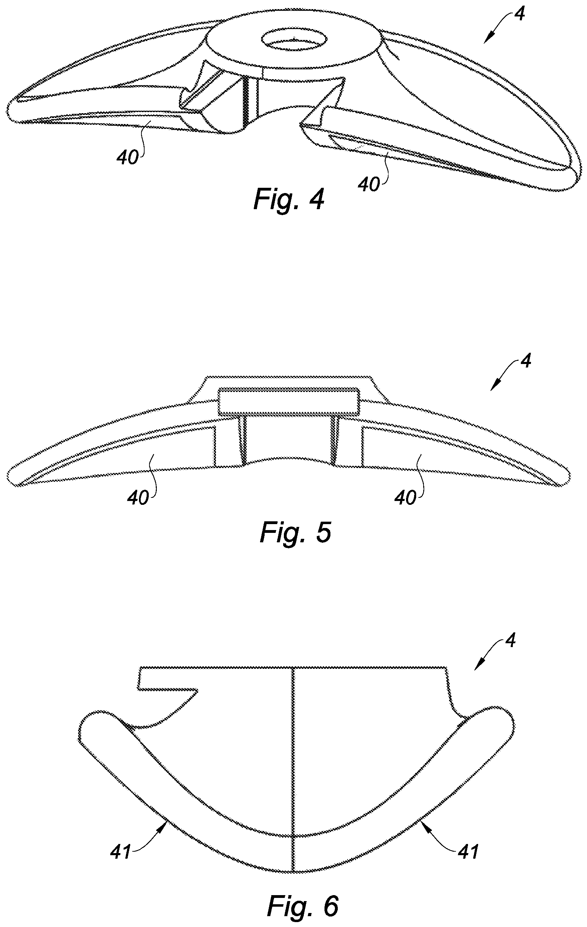

Les ailettes 4 de préhension s'étendent suivant un axe longitudinal, et présentent avantageusement un profil transversal formant une surface convexe 41 (bien visible à la figure 6).

Avantageusement, les ailettes 4 de préhension sont réalisées au moins partiellement dans le premier matériau plastique. Avantageusement, les ailettes 4 de préhension présentent une surface de préhension 40 au moins partiellement réalisée dans le second matériau plastique.

### Manipulation de la seringue

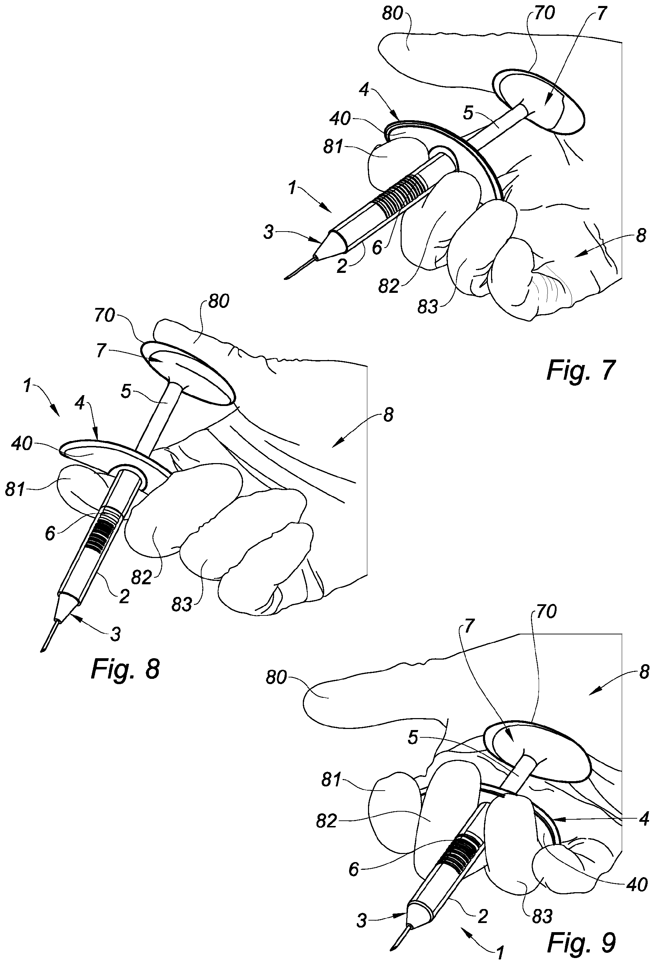

Comme illustré aux figures 7 à 9, différentes manipulations de la seringue 1 selon l'invention sont possibles, ce qui est notamment rendu possible par les aires importantes des surfaces de préhension 40, 70 des ailettes 4 de préhension et du poussoir 7, ainsi que par la position du centre de gravité de la seringue 1. Ces différentes manipulations de la seringue 1 se traduisent par des positions différentes de la seringue 1 dans la main 8 du médecin.

Dans la position illustrée à la figure 6, le corps 2 de seringue est saisi entre l'index 81 et le majeur 82 du médecin, reposant sur les ailettes 4 de préhension, tandis que le poussoir 7 est actionné par la base du pouce 80.

Dans la position illustrée à la figure 7, le corps 2 de seringue est saisi entre l'index 81 et le majeur 82 du médecin, reposant sur les ailettes 4 de préhension, tandis que le poussoir 7 est actionné par la pulpe du pouce 80.

Dans la position illustrée à la figure 8, le corps 2 de seringue est saisi entre le majeur 82 et l'annulaire 83 du médecin, reposant sur les ailettes 4 de préhension, tandis que le poussoir 7 est actionné par la paume de la main 8.

L'invention ne se limite pas aux modes de réalisation exposés. L'homme du métier est mis à même de considérer leurs combinaisons techniquement opérantes, et de leur substituer des équivalents.

## Revendications

1. Seringue (1), comportant :
- un corps (2) de seringue, destiné à recevoir un produit à injecter ; le corps (2) de seringue présentant une première extrémité (20), destinée à être équipée d'un porte-aiguille (3), et une seconde extrémité (21) opposée ;
- des ailettes (4) de préhension, agencées à la seconde extrémité (21) du corps (2) de seringue ;
- une tige (5) de piston, montée coulissante à l'intérieur du corps (2) de seringue, la tige (5) de piston présentant une première extrémité (50) et une seconde extrémité (51) opposée ;
- un butoir (6), agencé à la première extrémité (50) de la tige (5) de piston, et présentant une position de fin de course à l'intérieur du corps (2) de seringue ;
- un poussoir (7), agencé à la seconde extrémité (51) de la tige (5) de piston ;
le corps (2) de seringue, les ailettes (4) de préhension, la tige (5) de piston et le butoir (6) possédant une masse totale ;
la seringue (1) étant **caractérisée en ce que** le poussoir (7), sur lequel est exercée manuellement une pression adaptée pour injecter le produit à injecter, possède une masse supérieure ou égale à une valeur seuil, déterminée en fonction de la masse totale, et au-delà de laquelle la seringue (1) possède un centre de gravité situé à l'extérieur du corps (2) de seringue, au sein de la tige (5) de piston, entre les ailettes (4) de préhension et le poussoir (7), lorsque le butoir (6) est dans la position de fin de course.

2. Seringue (1) selon la revendication 1, dans laquelle le centre de gravité est situé à proximité de la seconde extrémité (51) de la tige (5) de piston lorsque le butoir (6) est dans la position de fin de course.

3. Seringue (1) selon la revendication 1 ou 2, dans laquelle la valeur seuil de la masse du poussoir (7) est 4 grammes.

4. Seringue (1) selon l'une des revendications 1 à 3, dans laquelle le poussoir (7) présente une surface de préhension (70), de préférence elliptique, possédant une aire comprise entre 800 mm² et 900 mm².

5. Seringue (1) selon l'une des revendications 1 à 4, dans laquelle les ailettes (4) de préhension présentent une surface de préhension (40) possédant une aire comprise entre 700 mm² et 800 mm², de préférence comprise entre 700 mm² et 750 mm².

6. Seringue (1) selon l'une des revendications 1 à 5, dans laquelle les ailettes (4) de préhension présentent une surface de préhension (40) concave, orientée vers la première extrémité (20) du corps (2) de seringue.

7. Seringue (1) selon l'une des revendications 1 à 6, dans laquelle les ailettes (4) de préhension s'étendent suivant un axe longitudinal, et présentent un profil transversal formant une surface convexe (41).

8. Seringue (1) selon l'une des revendications 1 à 7, dans laquelle le corps (2) de seringue, les ailettes (4) de préhension, la tige (5) de piston et le poussoir (7) sont réalisés au moins partiellement dans un premier matériau plastique, de préférence le polycarbonate ; seringue (1) dans laquelle le poussoir (7) présente une surface de préhension (70) au moins partiellement réalisée dans un second matériau plastique, différent du premier matériau plastique, et possédant une dureté comprise entre 30 Shore A et 70 Shore A, de préférence comprise entre 50 Shore A et 70 Shore A ; seringue (1) dans laquelle les ailettes (4) de préhension présentent une surface de préhension (70) au moins partiellement réalisée dans le second matériau plastique.

## Patentansprüche

1. Spritze (1), welche umfasst:
- einen Spritzenkörper (2), der dazu bestimmt ist, ein zu injizierendes Produkt aufzunehmen; wobei der Spritzenkörper (2) ein erstes Ende (20), das dazu bestimmt ist, mit einem Nadelträger (3) ausgestattet zu werden, und ein entgegengesetztes zweites Ende (21) aufweist;
- Griffstege (4), die am zweiten Ende (21) des Spritzenkörpers (2) angeordnet sind;
- eine Kolbenstange (5) die im Inneren des Spritzenkörpers (2) verschiebbar gelagert ist, wobei die Kolbenstange (5) ein erstes Ende (50) und ein entgegengesetztes zweites Ende (51) aufweist;
- einen Anschlag (6), der am ersten Ende (50) der Kolbenstange (5) angeordnet ist und eine Endlage im Inneren des Spritzenkörpers (2) aufweist;
- ein Druckstück (7), das am zweiten Ende (51) der Kolbenstange (5) angeordnet ist;
wobei der Spritzenkörper (2), die Griffstege (4), die Kolbenstange (5) und der Anschlag (6) eine Gesamtmasse besitzen;
wobei die Spritze (1) **dadurch gekennzeichnet ist, dass** das Druckstück (7), auf welches manuell ein Druck ausgeübt wird, der geeignet ist, das zu injizierende Produkt zu injizieren, eine Masse besitzt, die größer oder gleich einem Schwellenwert ist, der in Abhängigkeit von der Gesamtmasse bestimmt wird und bei dessen Überschreiten die Spritze (1) einen Schwerpunkt besitzt, der sich außerhalb des Spritzenkörpers (2), im Inneren der Kolbenstange (2), zwischen den Griffstegen (4) und dem Druckstück (7) befindet, wenn sich der Anschlag (6) in der Endlage befindet.

2. Spritze (1) nach Anspruch 1, wobei sich der Schwerpunkt in der Nähe des zweiten Endes (51) der Kolbenstange (5) befindet, wenn sich der Anschlag (6) in der Endlage befindet.

3. Spritze (1) nach Anspruch 1 oder 2, wobei der Schwellenwert der Masse des Druckstücks (7) 4 Gramm beträgt.

4. Spritze (1) nach einem der Ansprüche 1 bis 3, wobei das Druckstück (7) eine vorzugsweise elliptische Greiffläche (70) aufweist, die einen Flächeninhalt zwischen 800 mm² und 900 mm² besitzt.

5. Spritze (1) nach einem der Ansprüche 1 bis 4, wobei die Griffstege (4) eine Greiffläche (40) aufweisen, die einen Flächeninhalt zwischen 700 mm² und 800 mm², vorzugsweise zwischen 700 mm² und 750 mm² besitzt.

6. Spritze (1) nach einem der Ansprüche 1 bis 5, wobei die Griffstege (4) eine konkave Greiffläche (40) aufweisen, die dem ersten Ende (20) des Spritzenkörpers (2) zugewandt ist.

7. Spritze (1) nach einem der Ansprüche 1 bis 6, wobei sich die Griffstege (4) entlang einer Längsachse erstrecken und ein Querprofil aufweisen, das eine konvexe Fläche (41) bildet.

8. Spritze (1) nach einem der Ansprüche 1 bis 7, wobei der Spritzenkörper (2), die Griffstege (4), die Kolbenstange (5) und das Druckstück (7) wenigstens teilweise aus einem ersten Kunststoff hergestellt sind, vorzugsweise aus Polycarbonat; wobei das Druckstück (7) der Spritze (1) eine Greiffläche (70) aufweist, die wenigstens teilweise aus einem zweiten Kunststoff hergestellt ist, der von dem ersten Kunststoff verschieden ist und eine Härte zwischen 30 Shore A und 70 Shore A, vorzugsweise zwischen 50 Shore A und 70 Shore A besitzt; wobei die Griffstege (4) der Spritze (1) eine Greiffläche (70) aufweisen, die wenigstens teilweise aus dem zweiten Kunststoff hergestellt ist.

## Claims

1. Syringe (1), having:
- a syringe body (2), intended to receive a product for injection, the syringe body (2) having a first end (20), intended to be equipped with a needle holder (3), and a second, opposite end (21);
- gripping wings (4), arranged at the second end (21) of the syringe body (2);
- a piston rod (5), mounted so as to be able to slide inside the syringe body (2), the piston rod (5) having a first end (50) and a second, opposite end (51);
- a stop (6), arranged at the first end (50) of the piston rod (5), and having an end-of-travel position inside the syringe body (2);
- a pusher (7), arranged at the second end (51) of the piston rod (5);
the syringe body (2), the gripping wings (4), the piston rod (5) and the stop (6) having a total mass;
the syringe (1) being **characterized in that** the pusher (7), on which a pressure suitable for injecting the product for injection is manually exerted, has a mass greater than or equal to a threshold value determined as a function of the total mass, beyond which the syringe (1) has a centre of gravity situated outside the syringe body (2), inside the piston rod (5), between the gripping wings (4) and the pusher (7), when the stop (6) is in the end-of-travel position.

2. Syringe (1) according to Claim 1, in which the centre of gravity is situated in proximity to the second end (51) of the piston rod (5) when the stop (6) is in the end-of-travel position.

3. Syringe (1) according to Claim 1 or 2, in which the threshold value of the mass of the pusher (7) is 4 grams.

4. Syringe (1) according to one of Claims 1 to 3, in which the pusher (7) has a gripping surface (70), preferably of elliptical shape, having a surface area of between 800 mm² and 900 mm².

5. Syringe (1) according to one of Claims 1 to 4, in which the gripping wings (4) have a gripping surface (40) that has a surface area of between 700 mm² and 800 mm², preferably of between 700 mm² and 750 mm².

6. Syringe (1) according to one of Claims 1 to 5, in which the gripping wings (4) have a concave gripping surface (40) oriented towards the first end (20) of the syringe body (2).

7. Syringe (1) according to one of Claims 1 to 6, in which the gripping wings (4) extend along a longitudinal axis and have a transverse profile forming a convex surface (41).

8. Syringe (1) according to one of Claims 1 to 7, in which the syringe body (2), the gripping wings (4), the piston rod (5) and the pusher (7) are made at least partially of a first plastics material, preferably polycarbonate; in which syringe (1) the pusher (7) has a gripping surface (70) made at least partially of a second plastics material different than the first plastics material and having a hardness of between 30 Shore A and 70 Shore A, preferably of between 50 Shore A and 70 Shore A; in which syringe (1) the gripping wings (4) have a gripping surface (70) made at least partially of the second plastics material.
